# EUROPÄISCHE PATENTSCHRIFT

(11) **EP 1 997 942 B1**
(45) Veröffentlichungstag und Bekanntmachung des Hinweises auf die Patenterteilung: **30.06.2010**
(21) Anmeldenummer: 07010876.6
(22) Anmeldetag: 01.06.2007
(51) Int. Cl.: D04B 21/02, A44B 18/00, A61F 13/15

(54) **Verbundstoffelement für einen Klettverschluss**
Composite element for a Velcro fastener
Elément de liaison pour une fermeture à crochet

(43) Veröffentlichungstag der Anmeldung: 03.12.2008
(73) Patentinhaber: Nordenia Deutschland Gronau GmbH, 48599 Gronau (DE)
(72) Erfinder: Baldauf, Georg, 48366 Laer (DE); Homölle, Dieter, Ochtrup (DE)
(74) Vertreter: Albrecht, Rainer Harald

(56) Entgegenhaltungen:
- WO-A-2004/050970
- WO-A-2006/071211

## Beschreibung

Die Erfindung betrifft ein Verbundstoffelement für einen Klettverschluss, insbesondere einen Windelverschluss, bestehend aus einem bedruckten Träger und einer auf dem Träger aufkaschierten, textilen Deckschicht, die durch Wirken hergestellte freie Schlaufen für den Eingriff von Hakenelementen aufweist.

Das Verbundstoffelement bildet den weiblichen Teil eines Klettverschlusses. Bei der Verwendung an Windeln wird das Verbundstoffelement auf dem vorderen Bündchenbereich der Windel angebracht. Ein Verschlussband, das seitlich an der Windel befestigt ist und an seinem freien Ende Kletthaken aufweist, vervollständigt den Klettverschluss. Klettverschlüsse können mehrfach geöffnet und verschlossen werden, ohne dass dadurch die Funktionalität des Verschlusses leidet. Im Gegensatz zu Klebeverschlüssen sind Klettverschlüsse unempfindlich gegenüber Kontakt mit Hautcremes oder Puder.

An ein Verbundstoffelement für einen Klettverschluss an einem Wegwerfprodukt, z. B. Babywindeln, werden mehrere Anforderungen gestellt. Das textile Material soll ein möglichst geringes Flächengewicht aufweisen, damit es kostengünstig gefertigt werden kann. Es soll durchscheinend sein, damit die bedruckte Oberfläche des Trägers und auf den Träger gedruckte Rapportmarken für die weitere Verarbeitung des Verbundstoffes sichtbar sind. Trotz seines geringen Flächengewichtes muss das textile Material eine ausreichende Verhakung mit Kletthaken des zugeordneten Verschlussbandes gewährleisten. Erforderlich ist eine ausreichende Zahl von frei beweglichen Schlaufen und Fasern, deren Funktion durch eine Verklebung des Trägers mit der textilen Deckschicht nicht beeinträchtigt werden darf. Nicht zuletzt soll das Material eine den Verbraucher ansprechende Anmutung besitzen. Angestrebt werden optische und haptische Eigenschaften eines textilen Materials.

Ein Verbundstoffelement mit den eingangs beschriebenen Merkmalen und der eingangs erläuterten Zweckbestimmung ist aus der WO 2004/050970 A und der EP 1 690 967 A1 bekannt. Der Träger besteht aus einer ein- oder mehrschichtigen Kunststofffolie, wobei als Kunststoffe Polyolefine, Polyester, Polyamide, Mischungen und Copolymerisate dieser Polymere genannt sind. Die textile Deckschicht weist eine zur Verbindung mit Kletthaken geeignete Oberflächenstruktur auf und ist mit der Kunststofffolie verklebt. Durch einen nicht vollflächigen Klebstoffauftrag wird eine gute Klettwirkung erreicht, da Kletthaken sich in klebstofffreien Bereichen gut mit den oberflächlichen Schlaufen sowie den Fasern des textilen Materials verhaken können. Trotz einer Bedruckung des Folienträgers ist die Anmutung des Materials noch verbesserungsbedürftig. Durch die glänzende Oberfläche der Kunststofffolie geht die textile Wirkung des Materials verloren. Ferner ist die Folie relativ steif, so dass das Verbundstoffelement als Fremdkörper auf der Windeloberfläche wahrgenommen wird. Schließlich bildet das Verbundstoffelement luftundurchlässige Bereiche, die der Verbraucher ebenfalls als störend empfindet, da die umgebenden Bereiche der Windel oft atmungsaktiv ausgeführt sind.

Der Erfindung liegt die Aufgabe zugrunde, Haptik, visuelle Eigenschaften und insbesondere die Luftdurchlässigkeit des Verbundstoffelementes zu verbessern.

Ausgehend von einem Verbundstoffelement mit den eingangs beschriebenen Merkmalen wird die Aufgabe erfindungsgemäß dadurch gelöst, dass der Träger aus einem Nonwoven besteht, welches auf der zur Deckschicht zugewandten, innenliegenden Seite im Rotationsdruckverfahren bedruckt ist. Derartige Träger werden in der WO 2006/071211 A vorgeschlagen und in Verbindung mit einer als Nonwoven ausgebildeten Deckschicht dargestellt. Das Rotationsdruckverfahren zeichnet sich trotz vergleichsweise hoher Einrichtungskosten bei großen Druckmengen durch seine Wirtschaftlichkeit aus. So können mit dem Rotationsdruckverfahren sehr hohe Bahngeschwindigkeiten und so auch ein großer Durchsatz realisiert werden. Das Rotationsdruckverfahren erfolgt vorzugsweise durch eine Tiefdrucktechnik, wobei sowohl ein direkter als auch ein indirekter Tiefdruck durchgeführt werden kann. Bei einem direkten Tiefdruck wird die Farbe aus Vertiefungen des Druckzylinders, den Näpfchen, direkt auf das Substrat übertragen. Beim indirekten Tiefdruck wird die Farbe aus den Näpfchen zunächst auf einen typischerweise aus Gummi gefertigten Presseur und von dort nachfolgend auf das Nonwoven aufgebracht. Während mit dem indirekten Tiefdruck ein gleichmäßiger Farbauftrag erreicht werden kann, ermöglicht ein direkter Tiefdruck ohne weiteres auch den Auftrag größerer Farbmengen.

Unter Nonwoven werden flächige Faservliese verstanden. Der Begriff umfasst sowohl Stapelvliesware als auch Spinnvliese aus endlosen Filamentfasern. Aufgrund seiner höheren Reißfestigkeit ist ein Spinnvlies oder ein mehrlagiges Nonwoven-Material mit zumindest einer Außenlage aus Spinnvlies in besonderem Maße geeignet. Bevorzugt ist ein mehrlagiges Nonwoven, welches Außenschichten aus Spinnvlies (Spunbond S) und dazwischen zumindest eine Schicht aus schmelzgeblasenen Fasern (Meltblown M) aufweist. Diese mehrlagigen Nonwoven-Materialien mit einer Schichtstruktur SMS, SMMS oder SMMMS zeichnen sich üblicherweise gegenüber einem reinen Spinnvlies durch eine gleichmäßigere Oberflächenstruktur und somit eine bessere Bedruckbarkeit aus. Das Nonwoven kann aus Polyolefinfasern, Polyamidfasern, Polyesterfasern oder Fasermischungen der genannten Materialien bestehen. Das Flächengewicht des als Träger eingesetzten Nonwovens liegt zweckmäßig in einem Bereich zwischen 10 g/m² und 30 g/m². Der eingesetzte Spinnvliesstoff ist vorzugsweise ein SMS aus Polypropylen im Gewichtsbereich von 15 g/m².

Im Vergleich zu einem Klettverschlusselement, dessen Träger aus einer Kunststofffolie besteht, zeichnet sich das erfindungsgemäße Verbundstoffelement durch eine höhere Flexibilität, Weichheit und Atmungsaktivität aus. Die bedruckte Oberfläche des Nonwovens scheint durch die relativ transparente textile Deckschicht, die durch Wirken hergestellte freie Schlaufen für den Eingriff von Hakenelementen aufweist, hindurch. Der Aufdruck ist durch die textile Deckschicht vor Abrieb geschützt. Das Material hat das Aussehen und die Oberflächeneigenschaften eines bedruckten, textilen Stoffes. Darüber hinaus wirkt sich der aus einem Nonwoven bestehende Träger positiv auf die Klettwirkung der in das Material eintauchenden Hakenelemente aus, da die Hakenelemente auch mit Faseranteilen des Nonwovens in Wechselwirkung treten können.

Das Bedrucken von nicht gewebten Textilmaterialien (Nonwoven) ist bekannt und bei Zellulosesubstraten unproblematisch. Schwieriger ist das Bedrucken von Nonwoven aus Polyamidfasern, Polyesterfasern und vor allem Polyolefinfasern. Insbesondere auf faserartigen Polyolefinstrukturen weisen konventionell benutzte Druckfarben und Farbstoffe nur eine begrenzte Adhäsion auf, was sich nachteilig auf die Wisch- und Abriebsfestigkeit eines Druckbildes auswirkt. Eine geringere Wisch- und Abriebsfestigkeit kann bei dem erfindungsgemäßen Verbundstoffelement in Kauf genommen werden, da die oberseitige textile Deckschicht, die durch Wirken hergestellte freie Schlaufen für den Eingriff von Hakenelementen aufweist und selbst nicht bedruckt ist, das Druckbild schützt. Das Druckbild ist auf der zur Deckschicht zugewandten Seite angeordnet und wird unterseitig von dem Nonwoven und oberseitig von der textilen Deckschicht geschützt.

Um die Bedruckbarkeit und die Qualität des Druckbildes zu verbessern, kann die zu bedruckende Oberfläche des Nonwovens vorbehandelt werden. Zusätzlich soll bei dem Rotationsdruckverfahren ein Durchschlagen der Druckfarbe durch das Nonwoven vermieden werden, da ansonsten die Walzen der Druckmaschine verschmutzen können. Abgesehen davon, dass derartige Verschmutzungen eine aufwendige Reinigung erfordern, können antrocknende Farbreste auch zu einem Zerfasern oder sogar zu einer Zerstörung des Nonwoven beitragen, so dass insbesondere auch zur Vermeidung eines Durchschlagens der Druckfarbe eine Vorbehandlung der zu bedruckenden Oberfläche des Nonwovens zweckmäßig ist. Schließlich ist auch ein Durchschlagen eines Kaschierklebers bei der Verbindung des Nonwovens mit der Deckschicht zu vermeiden. Es bieten sich verschiedene Möglichkeiten einer Vorbehandlung an. Eine erste Ausgestaltung sieht eine Vorbehandlung der Oberfläche des Nonwovens mit einem thixotropen Primer vor. Der Primer ist eine Schicht, welche die Druckfarbe fixiert und in einem geeigneten Bindemittel anorganische Füllstoffe, z. B. Siliziumdioxid, Titandioxid, Calciumcarbonate, calcinierten Ton oder dergleichen zur Verbesserung der Absorption und Haftung der Druckfarbe enthält. Der thixotrop eingestellte Primer verhält sich unter Scherspannung wie eine Flüssigkeit und lässt sich aufgrund seiner thixotropen Eigenschaften als dünner Film auf die Faseroberfläche des Nonwovens auftragen. Ohne Scherbeanspruchung hat der Primer die Eigenschaften eines Feststoffes, der an der Faseroberfläche haftet. Die thixotropen Eigenschaften erleichtern das Aufbringen des Primers. Für den Primer können die für Druckfarben üblichen Bindemittel wie beispielsweise Nitrocellulose (NC), Polyvinylbutyral (PVB) oder Polyvinylchlorid (PVC) eingesetzt werden. Es versteht sich, dass der Primer als Bindemittel auch Harze enthalten kann, die beispielsweise mit einem bei niedrigen Temperaturen aushärtenden Reaktionsmittel vernetzen. Der Primer kann in einer Menge zwischen 0,1 g/m² und 20 g/m², vorzugsweise zwischen 0,5 g/m² und 2 g/m², aufgetragen werden.

Gemäß einer weiteren Ausführungsvariante der Erfindung weist die zu bedruckende Oberfläche des Nonwovens eine durch Sprühen aufgebrachte Schicht auf, die eine im Wesentlichen geschlossene Haut bildet und vorzugsweise eine Schichtdicke von weniger als 5 µm aufweist. Auf der geschlossenen Oberfläche der aufgesprühten Schicht kann ein qualitativ hochwertiges Druckbild erzeugt werden, da die Schicht ein Durchschlagen der Druckfarbe verhindert.

Auf der zu bedruckenden Oberfläche des Nonwovens kann ferner eine feinporöse Schicht zur Absorption der Druckfarbe aufgebracht sein. Feinporöse Beschichtungen sind als so genannte "Foamcoatings" bekannt und werden beispielsweise zur Appretur von Baumwolltextilien eingesetzt. Die erfindungsgemäße Vorbehandlung der Nonwovenschichten mit einer feinporösen Schicht verbessert die Bedruckbarkeit der nicht gewebten Schichten, insbesondere wenn diese aus polyolefinischen Fasern bestehen. Es kann auf entsprechend vorbehandelten Nonwovenschichten ein besseres Druckbild erzeugt werden, wobei gleichzeitig die Abriebsfestigkeit und Wischfestigkeit verbessert wird.

Im Rahmen der Erfindung kann auch vorgesehen sein, dass die zu bedruckende Oberfläche des Nonwovens zur Vorbereitung mit einer durch eine Breitschlitzextrusionsdüse aufgetragenen Schicht versehen wird. Bei einem solchen so genannten Curtain-Coating-Verfahren durchläuft das Nonwoven einen Schmelzvorhang, der aus der Breitschlitzdüse austritt.

Zusätzlich oder alternativ zu den beschriebenen Möglichkeiten der Vorbehandlung ist das Nonwoven an seiner bedruckten Seite vorzugsweise durch eine Corona-Entladung vorbehandelt. Durch eine solche Behandlung kann die Oberflächenstruktur der Fasern des Nonwoven derart modifiziert werden, dass diese leichter bedruckt oder mit einer zusätzlichen Beschichtung versehen werden können. Das Nonwoven kann auch mit einem Plasmaprozess, insbesondere einem Plasmabeschichtungsprozess, vorbehandelt werden: Geeignet sind insbesondere Plasmapolymerisationsprozesse, bei denen die Oberfläche durch eine Abscheidung bestimmter Materialien aus dem Plasma modifiziert werden. Aus prozesstechnischen Gründen werden vorzugsweise Plasmaverfahren eingesetzt, die bei Atmosphärendruck durchgeführt werden können, da dabei eine aufwendige Vakuumanordnung, durch die das Nonwoven geführt wird, nicht erforderlich ist.

Die Deckschicht des erfindungsgemäßen Verbundstoffelementes besteht vorzugsweise aus einer Wirkware, die Schussfäden und wirktechnisch hergestellte Kettstränge mit darin eingebundenen Schlaufen aufweist. Die Deckschicht ist mit dem aus Nonwoven bestehenden Träger verklebt, wobei der Klebstoff zweckmäßig in einem Muster aufgetragen wird, welches sich aus Klebeflächen und klebstofffreien Flächen zusammensetzt. Geeignet sind insbesondere die in EP 1 690 967 A1 beschriebenen Klebemuster. Der Auftrag des Klebstoffes erfolgt zweckmäßig im Rotationsdruckverfahren. Die Verklebung kann mit reaktiven PUR Klebstoffen oder Schmelzeklebern erfolgen. Der Klebstoffauftrag erfolgt vorzugsweise in Form von Streifen- oder Punktmustern. Dabei beträgt die verklebte Fläche 10 bis 70 % vorzugsweise 60 bis 40 %. Der Anteil der verklebten Fläche sowie Art und Form des Klebstoffauftrages richten sich nach den typischen Öffnungskräften bei der Verwendung des Verbundstoffelementes in Kombination mit einem geeigneten Klettband. Bevorzugt bildet der applizierte Klebstoff von Klebstoffrahmen umschlossene Zellenmuster. Als besonders geeignete Klebstoffauftragsmuster haben sich neben Gittermustern, Streifen- und Punktmuster bewährt. Entscheidend ist jeweils, dass die textile Deckschicht nicht vollständig auf das Nonwoven geklebt wird, sondern in den unverklebten Bereichen offene, unverklebte Zellen aufweist.

Im Rahmen einer bevorzugten Weiterbildung entspricht die von einem der Klebstoffrahmen umschlossenen Abschnittslänge des Kaschierverbundes der Abschnittslänge des Windelverschlusses. Die rapportgenaue Ablängung und Applikation auf die Windel wird durch Ansteuerung an eine auf den Verbundstoff gedruckte Steuermarke erreicht. Die Rapportmarke kann sichtbar im Motiv gedruckt werden. Die Rapportmarke kann ebenso unsichtbar, d. h. nur mit unter UV-Licht sichtbarer Farbe gedruckt werden.

Durch die komplette Randverklebung jedes einzelnen Windelverschlusses wird ein Ausfransen der textilen Deckschicht im Gebrauch mit dem Kletthaken verhindert. Das Ausfransen, also das Abreißen der textilen Deckschicht von dem Träger und damit das Zerreißen einzelner Fasern und Filamente ist ein unerwünschter Nebeneffekt von teilweise verklebten Verbundstoffen für mechanische Windelverschlüsse. Dadurch, dass sich die Kletthaken in den nicht verklebten, offenen Zellen des Textils intensiver verhaken können als in den verklebten Bereichen, kann es beim Lösen der Klettverbindung zum Ausreißen des Textils vor allen in den Randbereichen des Windelverschlusses kommen. Durch die rahmenförmige Verklebung des Randbereiches wird das Ausfransen verhindert.

Ein vollflächiger Klebstoffauftrag ist ebenfalls möglich. Bei niedrigeren Öffnungskräften aber hohen Anforderungen an die Verbundhaftung ist die vollflächige Verklebung einer teilweisen Verklebung vorzuziehen.

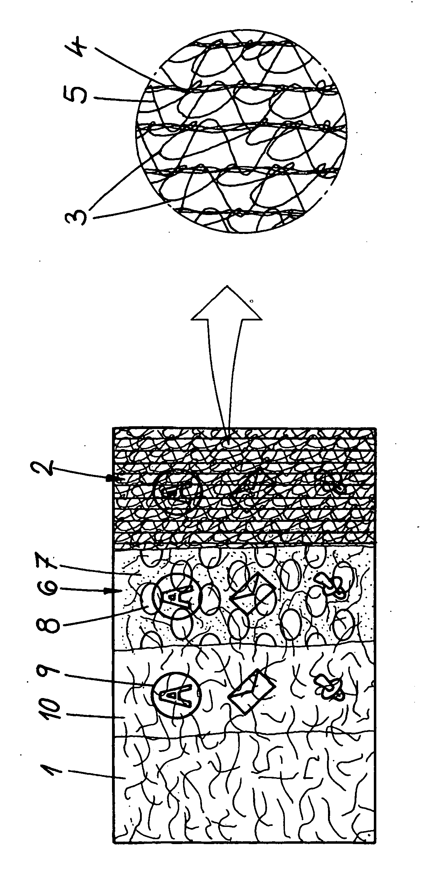

Im Folgenden wird die Erfindung anhand einer lediglich ein Ausführungsbeispiel darstellenden Zeichnung erläutert. Die einzige Figur zeigt schematisch in einer Draufsicht den Schichtenaufbau auf ein erfindungsgemäßes Verbundstoffelement.

Das in der Figur dargestellte Verbundstoffelement bildet den weiblichen Teil eines Klettverschlusses und ist bei der Verwendung als Windelverschluss beispielsweise auf dem vorderen Bündchenbereich einer Windel angebracht. Das Verbundstoffelement besteht aus einem Träger 1 und einer auf dem Träger aufkaschierten textilen Deckschicht 2, die freie Schlaufen 3 für den Eingriff von Hakenelementen aufweist. Die Deckschicht 2 besteht aus einer Wirkware und weist wirktechnisch hergestellte Kettstränge 4 mit darin eingebundenen Schlaufen 3 sowie Schussfäden 5 auf. Die Deckschicht 2 ist mit dem Träger 1 verklebt, wobei der Klebstoff 6 in einem Muster aufgetragen ist, das sich aus Klebeflächen 7 und klebstofffreien Flächen 8 zusammensetzt. Im Ausführungsbeispiel weist der Klebstoffauftrag ein Muster mit einer zellenförmigen Struktur auf.

Der Träger 1 besteht aus Nonwoven, vorzugsweise einem Spinnvlies aus endlosen Filamentfasern, und ist auf der zur Deckschicht zugewandten, innenliegenden Seite im Rotationsdruckverfahren bedruckt. Die bedruckte Oberfläche 9 des Nonwovens ist durch die offene Struktur der textilen Deckschicht 2 hindurch sichtbar.

Das den Träger 1 bildende Nonwoven besteht aus polyolefinischen Fasern, vorzugsweise Polypropylenfasern. Dieses Material kann ohne Vorbehandlung nur mit speziellen, lösemittelhaltigen Farben bedruckt werden. Um die Bedruckbarkeit zu verbessern, ist die zu bedruckende Oberfläche des Nonwovens vorbehandelt worden. Dargestellt wurde die Vorbehandlung in Form einer Schicht 10, die auf verschiedene Weise herstellbar ist. Im Rahmen der Erfindung liegt es, dass die Oberfläche des Nonwovens mit einem thixotropen Primer vorbehandelt wird, der die Druckfarbe fixiert und neben einem geeigneten Bindemittel anorganische Füllstoffe, z. B. Siliziumdioxid, Titandioxid, Calciumcarbonate, calcinierten Ton oder dergleichen zur Verbesserung der Haftung der Druckfarbe enthält. Die Schicht 10 kann ferner aus einer durch Sprühen auf das Nonwoven aufgetragenen Schicht bestehen, die eine im Wesentlichen geschlossene Haut auf dem Nowoven bildet und eine geringe Schichtdicke von beispielsweise weniger als 5 µm aufweist. Schließlich kann die Vorbehandlung darin bestehen, dass auf der zu bedruckenden Oberfläche des Nonwovens eine feinporöse Schicht zur Absorption der Druckfarbe aufgebracht ist.

## Patentansprüche

1. Verbundstoffelement für einen Klettverschluss, insbesondere einen Windelverschluss, bestehend aus einem bedruckten Träger (1) und einer auf dem Träger (1) aufkaschierten, textilen Deckschicht (2), die durch Wirken hergestellte freie Schlaufen (3) für den Eingriff von Hakenelementen aufweist, **dadurch gekennzeichnet, dass** der Träger (1) aus einem Non-woven besteht, welches auf der zur Deckschicht zugewandten, innenliegenden Seite im Rotationsdruckverfahren bedruckt ist.

2. Verbundstoffelement nach Anspruch 1, **dadurch gekennzeichnet, dass** das Nonwoven (1) aus einem Spinnvlies besteht.

3. Verbundstoffelement nach Anspruch 1, **dadurch gekennzeichnet, dass** das Nonwoven (1) mehrlagig ist und zumindest eine Außenlage aus Spinnvlies aufweist.

4. Verbundstoffelement nach einem der Ansprüche 1 bis 3, **dadurch gekennzeichnet, dass** das Nonwoven (1) ein Flächengewicht von 10 bis 30 g/m² aufweist.

5. Verbundstoffelement nach einem der Ansprüche 1 bis 4, **dadurch gekennzeichnet, dass** die Druckfarbe auf einer mit einem thixotropen Primer vorbehandelten Oberfläche (10) des Nonwovens (1) aufgebracht ist.

6. Verbundstoffelement nach einem der Ansprüche 1 bis 4, **dadurch gekennzeichnet, dass** die Druckfarbe auf eine durch Sprühen auf das Nonwoven (1) aufgetragene Schicht (10) aufgebracht ist, wobei die Schicht (10) eine im Wesentlichen geschlossene Haut auf dem Nonwoven (1) bildet und eine Schichtdicke von weniger als 5 µm aufweist.

7. Verbundstoffelement nach einem der Ansprüche 1 bis 4, **dadurch gekennzeichnet, dass** die Druckfarbe auf eine durch eine Breitschlitzextrusionsdüse aufgetragene Schicht (10) aufgebracht ist.

8. Verbundstoffelement nach einem der Ansprüche 1 bis 4, **dadurch gekennzeichnet, dass** auf der zu bedruckenden Oberfläche des Nonwovens (1) eine feinporöse Schicht zur Absorption der Druckfarbe aufgebracht ist.

9. Verbundstoffelement nach einem der Ansprüche 1 bis 8, **dadurch gekennzeichnet, dass** das Nonwoven (1) an seiner bedruckten Seite durch eine CoronaEntladung oder einen Plasmaprozess, insbesondere einen Plasmabeschichtungsprozess, vorbehandelt ist.

10. Verbundstoffelement nach einem der Ansprüche 1 bis 9, **dadurch gekennzeichnet, dass** die Deckschicht (2) aus einer Wirkware besteht, die Schussfäden (5) und wirktechnisch hergestellte Kettstränge (4) mit darin eingebundenen Schlaufen (3) aufweist.

11. Verbundstoffelement nach einem der Ansprüche 1 bis 10, **dadurch gekennzeichnet, dass** die Deckschicht (2) mit dem Träger (1) verklebt ist und dass der Klebstoff in einem Muster aufgetragen ist, das sich aus Klebeflächen (7) und klebstofffreien Flächen (8) zusammensetzt.

## Claims

1. A composite material for a hook-and-loop fastener, in particular a diaper fastener, consisting of an imprinted support (1) and a textile cover layer (2), which is laminated onto the support (1) and which encompasses free loops (3), which are produced by means of knitting for engaging with hook elements, **characterized in that** the support (1) consists of a non-woven, which is imprinted on the inner side facing the cover layer by means of a rotary printing process.

2. The composite material according to claim 1, **characterized in that** the non-woven (1) consists of a spunbond fabric.

3. The composite material according to claim 1, **characterized in that** the non-woven (1) is multilayered and encompasses at least one outer layer made of spunbond fabric.

4. The composite material according to one of claims 1 to 3, **characterized in that** the non-woven (1) encompasses a mass per unit area of 10 to 30 g/m².

5. The composite material according to one of claims 1 to 4, **characterized in that** the printing ink is applied onto a surface (10) of the non-woven (1), which is pretreated with a thixotropic primer.

6. The composite material according to one of claims 1 to 4, **characterized in that** the printing ink is applied onto a layer (10), which is applied onto the non-woven (1) by means of spraying, wherein the layer (10) forms a substantially closed skin on the non-woven (1) and encompasses a layer thickness of less than 5 µm.

7. The composite material according to one of claims 1 to 4, **characterized in that** the printing ink is applied onto a layer (10), which is applied by means of a flat sheet extrusion nozzle.

8. The composite material according to one of claims 1 to 4, **characterized in that** a fine porous layer is applied onto the surface of the non-woven (1), which is to be imprinted, for absorbing the printing ink.

9. The composite material according to one of claims 1 to 8, **characterized in that** the non-woven (1) is pretreated on its imprinted side by means of a corona discharge or a plasma process, in particular a plasma coating process.

10. The composite material according to one of claims 1 to 9, **characterized in that** the cover layer (2) consists of a knit fabric, which encompasses weft yarns (5), and warp skeins (4), which are produced by means of knitting and which comprise loops (3), which are incorporated therein.

11. The composite material according to one of claims 1 to 10, **characterized in that** the cover layer (2) is adhered to the support (1) and **in that** the adhesive is applied in a pattern, which consists of adhesive surfaces (7) and adhesive-free surfaces (8).

## Revendications

1. Elément en matériau composite pour une fermeture auto-agrippante, notamment une fermeture d'une couche-culotte, consistant dans un support imprimé (1) et dans une couche de recouvrement textile (2) contrecollée sur le support (1), qui présente des boucles libres (3) produites par tricotage, pour l'engagement d'éléments à crochets, **caractérisé en ce que** le support (1) consiste dans un non-tissé, qui est imprimé par procédé d'impression par rotative sur la face interne, tournée vers la couche de recouvrement.

2. Elément en matériau composite selon la revendication 1, **caractérisé en ce que** le non-tissé (1) consiste dans un filé-lié.

3. Elément en matériau composite selon la revendication 1, **caractérisé en ce que** le non-tissé (1) est multicouches et présente au moins une couche extérieure en filé-lié.

4. Elément en matériau composite selon l'une quelconque des revendications 1 à 3, **caractérisé en ce que** le non-tissé (1) présente un grammage de 10 à 30 g/m².

5. Elément en matériau composite selon l'une quelconque des revendications 1 à 4, **caractérisé en ce que** l'encre d'impression est appliquée sur une surface (10) du non-tissé (1) qui est prétraitée avec une couche de fond thixotrope.

6. Elément en matériau composite selon l'une quelconque des revendications 1 à 4, **caractérisé en ce que** l'encre d'impression est appliquée sur une couche (10) posée par pulvérisation sur le non-tissé (1), la couche (10) formant une pellicule sensiblement close sur le non-tissé (1) et présentant une épaisseur de couche inférieure à 5 µm.

7. Elément en matériau composite selon l'une quelconque des revendications 1 à 4, **caractérisé en ce que** l'encre d'impression est appliquée sur une couche (10) posée à l'aide d'une filière d'extrusion plate.

8. Elément en matériau composite selon l'une quelconque des revendications 1 à 4, **caractérisé en ce que** sur la surface à imprimer du non-tissé (1) est posée une couche à micropores pour l'absorption de l'encre d'impression.

9. Elément en matériau composite selon l'une quelconque des revendications 1 à 8, **caractérisée en ce que** le non-tissé (1) est prétraité sur sa face imprimée par corona ou par processus plasma, notamment par processus de revêtement par plasma.

10. Elément en matériau composite selon l'une quelconque des revendications 1 à 9, **caractérisé en ce que** la couche de recouvrement (2) consiste dans un tricot qui présente des fils de trame (5) et des cordons de chaîne (4) produits par technique de tricotage, avec des boucles (3) liées dans ces derniers.

11. Elément en matériau composite selon l'une quelconque des revendications 1 à 10, **caractérisé en ce que** la couche de recouvrement (2) est collée sur le support (1) et que l'adhésif est appliqué selon un motif se composant de surfaces de collage (7) et de surfaces exemptes d'adhésif (8).
